# EUROPÄISCHE PATENTSCHRIFT

(11) **EP 0 727 494 B1**
(45) Veröffentlichungstag und Bekanntmachung des Hinweises auf die Patenterteilung: **30.09.1998**
(21) Anmeldenummer: 96102167.2
(22) Anmeldetag: 14.02.1996
(51) Int. Cl.: C12P 41/00

(54) **Verfahren zu enzymatischen Acylierung von Alkoholen mit Alkoxyvinylacetaten durch Umesterung**
Method for the enzymatic acylation of alcohols with alkoxyvinylacetates through transesterification
Méthode pour l'acylation enzymatique d'alcools avec des acétates d'alkoxyvinyl par transestérification

(30) Priorität: 20.02.1995 DE 19505672
(43) Veröffentlichungstag der Anmeldung: 21.08.1996
(73) Patentinhaber: HOECHST AKTIENGESELLSCHAFT, 65929 Frankfurt am Main (DE)
(72) Erfinder: Schudok, Manfred, 65795 Hattersheim (DE); Kretzschmar, Gerhard, Dr., 65760 Eschborn (DE)

(56) Entgegenhaltungen:
- EP-A- 0 321 918
- EP-A- 0 577 446
- JOURNAL OF THE CHEMICAL SOCIETY, PERKIN TRANSACTIONS 1, Nr. 23, 7.Dezember 1993, LETCHWORTH GB, Seiten 2999-3005, XP002003902 YASUYUKI KITA ET AL.: "Novel efficient synthesis of 1-Ethoxyvinyl esters using Ruthenium catalysts and their use in acylation of amines and alcohols: Synthesis of hydrophilic 3'-N-acylated Oxaunomycin derivatives"

## Beschreibung

Die Erfindung betrifft ein Verfahren zur enzymatischen Acylierung von primären und sekundären Alkoholen mit Enolestern, insbesondere Alkoxyvinylacetaten, in organischen Medien, wobei der Enolester in einen nach Enolisierung entstehenden einfachen Ester sowie einen Acylrest gespalten wird; dieser wird auf den sich in der Lösung befindlichen Alkohol übertragen, wobei die Übertragung bei racemischen bzw. prochiralen Alkoholen enantioselektiv verläuft, d. h. es findet selektive Acylierung nur des einen Enantiomeren bzw. pro-Enantiomeren statt, unter Zurücklassung des freien Alkohols des anderen Enantiomeren bzw. pro-Enantiomeren. Die chiralen acylierten Verbindungen und nicht umgesetzten chiralen Alkohole lassen sich einfach voneinander trennen. Aus den acylierten Alkoholen lassen sich gegebenenfalls die entsprechenden freien Alkohole durch einfache Esterspaltung gewinnen.

Viele optisch aktive Alkohole sind wichtige chirale Vorstufen von biologisch aktiven Substanzen wie Arzneistoffen, Naturstoffen, Pflanzenschutzmitteln und Flüssigkristallbausteinen; ein wirtschaftliches Herstellungsverfahren zur Acylierung chiraler, prochiraler aber manchmal auch nicht-chiraler Alkohole ist deshalb von großer Bedeutung.

Einige pharmakologische Wirkstoffe, deren Darstellung durch das erfindungsgemäße Verfahren erleichtert wird, sind beispielsweise NSAIDs (non-steroidal antiinflammatory drugs) wie Ibuprofen und Naproxen, Betablocker wie Nifenalol und Penbutolol, Bronchospasmolytica wie Tolubuterol und Bitolterol, Antimycotica wie Tioconazol, Pyrethroide wie Allethrine, darüberhinaus Tetramisol, Tetrahydrozolin, (R)-Tomoxetine und (S)-Fluoxetine, Prostaglandine, modifizierte und unmodifizierte Kohlenhydrate, z. B. Glucale, sowie Renin- und sonstige Proteaseinhibitoren, z. B. der HIV-Protease.

Außerdem ist die Acylierung mittels Enzymkatalyse im Gegensatz zur chemischen Acylierung, wo unerwünschte Nebenreaktionen auftreten können, durch ihre extrem milden Reaktionsbedingungen für besonders empfindliche primäre und sekundäre Alkohole von Bedeutung.

Es ist bekannt, daß man Vinylester zur enzymkatalysierten Veresterung von Alkoholen einsetzen kann. Beispielsweise wurde Schweinepankreas-Lipase verwendet und keine Stereoselektivität beobachtet (M. Degueil-Castaing et al., Tetrahedron Lett. 28, 953-954 (1987)).

Es ist außerdem bekannt, daß die enzymatische Racematspaltung von racemischen Alkoholen aufgrund einer selektiven enzymkatalysierten Umesterungsreaktion mit Vinylestern durchgeführt werden kann. Als Enzyme wurden Lipasen aus Schweineleber und -pankreas sowie verschiedenen Mikroorganismen verwendet, als Vinylester bevorzugt Vinylacetat (EP 0 321 918 A2). Beobachtet werden vielfach sehr hohe Enantiomerenüberschüsse bei der Verwendung racemischer Alkohole. Die nichtenzymatische Acylierung durch Umsetzung von Alkoholen mit Vinylestern ohne Enzymkatalyse gelingt nicht.

Weiterhin ist bekannt, daß für die enzymkatalysierten Umesterungsreaktionen mit Vinylacetat und anderen Vinylestern immobilisierte Enzyme eingesetzt werden können (EP 0 321 918 A2; EP 0 492 497 bzw. CA 2 058 185; EP 0 507 278 bzw. CA 2064 676). Der Einsatz der immobilisierten Enzyme zeichnete sich insbesondere durch eine erhöhte Langzeitstabilität aus.

Es ist bekannt, daß bei der Acylierung mit Vinylestern Acetaldehyd freigesetzt wird, der nach Enolisierung des primär entstehenden Vinylalkohols gebildet wird. Reaktive Aldehyde wie Acetaldehyd sind jedoch nicht inert, sondern können mit vielen funktionellen Gruppen Reaktionen eingehen; besonders leicht gelingt es zum Beispiel, Aminogruppen in Schiffsche Basen zu überführen (R. C. Larock, Comprehensive Organic Transformations, Verlag Chemie, New York, 1989, S. 398, 425, 426, 758, 759, 932). Diese bekannte Reaktion kann auch zur irreversiblen Modifizierung von Proteinen verwendet werden (V. V. Mozhaev, K. Martinek, Advanced Drug Delivery Reviews 4, 387-419 (1990)). Demnach wird das eingesetzte Enzym an den vorhandenen Aminogruppen, z. B. den Lysin-Seitenketten, modifiziert und kann dadurch schließlich auch desaktiviert werden. Dieser Inaktivierungsprozeß ist dadurch erkennbar, daß zum Beispiel rascher Aktivitätsverlust der freien Enzyme bei mehrfacher Verwendung in der Umesterungsreaktion mit Vinylacetat zu verzeichnen ist, im Gegensatz zur Verwendung der immobilisierten Enzyme, die eine zusätzliche Stabilisierung durch die polymere Matrix der Träger erfahren (EP 0 507 278 bzw. CA 2 064 676). Eine ebenfalls bekannte Reaktion der Aldehyde ist die unter bestimmten Bedingungen eintretende Polymerisierung bzw. Vernetzung, sowie auch die Halbacetal- und Acetalbildung

Der Fachmann erkennt somit sofort, daß Verfahren, die keinerlei reaktive Komponenten bei der enzymatischen Reaktion freisetzen, von Vorteil sind, da in diesem Fall keine Modifizierungen des Enzyms mit unerwünschten Auswirkungen sowie ebenfalls auch keine weiteren chemischen Nebenreaktion möglich sind.

Enolester, wie beispielsweise Ethoxyvinylacetat, sowie Verfahren zu deren Herstellung sind bekannt (Y. Kita, H. Maeda, K. Omori, T. Okuno, Y. Tamura, J. Chem. Soc. Perkin Trans. 1, 1993, S. 2999-3005; Y. Kita, H. Maeda, K. Omori, T. Okuno, Y. Tamura, Synlett 1993, S. 273-4; C. Bruneau, M. Neveux, Z. Kabouche, C. Ruppin, P. H. Dixneuf, Synlett 1993, S. 755-763). Ein Ausgangsprodukt zur Darstellung des Ethoxyvinylacetats ist Ethoxyacetylen.

Ethoxyacetylen oder das verwandte Methoxyacetylen kann beispielsweise auch als Kupplungsreagenz in der Peptidchemie eingesetzt werden (J. F. Arens, Recueil Chim. Pays Bas 74, 769-770 (1955)). Bekannt ist ebenfalls, daß bei dieser Aktivierung der Carboxylkomponente mit Ethoxyacetylen oder Methoxyacetylen in situ der Enolester gebildet wird dieser mit Nucleophilen wie z. B. Aminen oder Aminosäuren unter Freisetzung von Essigsäureethylester abreagiert. Für diese Reaktion ist aufgrund der starken Aktivierung der Carboxylgruppe durch den Enolester die Gegenwart eines Enzyms oder anderen Katalysators nicht erforderlich. Ebenso gelingt die Aminolyse verschiedener isolierter Enolester sehr leicht und ohne Katalysator (Y. Kita, H. Maeda, K. Omori, T. Okuno, Y. Tamura, J. Chem. Soc. Perkin Trans. 1, 1993, S. 2999-3005; Y. Kita, H. Maeda, K. Omori, T. Okuno, Y. Tamura, Synlett 1993, S. 273-4; Z. Kabouche, C. Bruneau, P. H. Dixneuf, Tetrahedron Lett. 32, 5359-62 (1991); C. Bruneau, M. Neveux, Z. Kabouche, C. Ruppin, P. H. Dixneuf, Synlett 1993, S. 755-763). Es gelingt jedoch nicht die einfache Alkoholyse der Enolester im Sinne einer Umesterungsreaktion. Dies ist nur dann möglich, wenn als Katalysator eine starke Säure wie Schwefelsäure oder p-Toluolsulfonsäure zugesetzt wird (Y. Kita, H. Maeda, K. Omori, T. Okuno, Y. Tamura, J. Chem. Soc. Perkin Trans. 1, 1993, S. 2999-3005; Y. Kita, H. Maeda, K. Omori, T. Okuno, Y. Tamura, Synlett 1993, S. 273-4; C. Bruneau, M. Neveux, Z. Kabouche, C. Ruppin, P. H. Dixneuf, Synlett 1993, S. 755-763).

Nach der Umsetzung mit Nucleophilen werden im Gegensatz zu den oben angeführten Verfahren mit Vinylestern keinerlei reaktive Verbindungen (wie Aldehyde) freigesetzt. Es entstehen lediglich einfache Ester wie z. B. Ethylacetat, bei denen es sich um bekanntermaßen unproblematische Standardlösemittel handelt, die selbst als Lösemittel für Reaktionen und enzymatische Umsetzungen eingesetzt werden, ohne selbst als Acyldonor zu fungieren.

Überraschenderweise wurde nun gefunden, daß es möglich ist, Enolester wie Ethoxyvinylacetat als Acyldonoren zur enzymkatalysierten O-Acylierung von primären und sekundären Alkoholen einzusetzen, ohne daß Blindreaktionen im Sinne einer nichtenzymatischen Acylierung eintreten. Gleichzeitig ist es auch möglich, im Fall von racemischen und prochiralen Alkoholen eine hervorragende Stereodifferenzierung im Sinne einer enantioselektiven Acylierung zu erhalten.

Die vorliegende Erfindung betrifft demgemäß ein Verfahren zur enzymatischen Acylierung von Alkoholen, das sich dadurch auszeichnet, daß man einen Ester der Formel I, 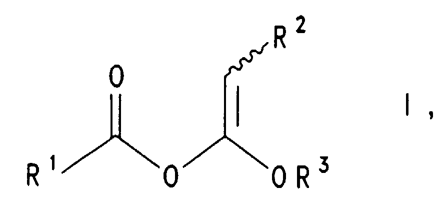 in der
- R¹: Wasserstoff, C₁-C₁₈-Alkyl, welches gegebenenfalls mit Halogen substituiert ist, Phenyl oder (C₁-C₃)-Alkoxy (C₁-C₄)-Alkyl und
- R²: Wasserstoff oder Methyl, wobei für den Fall, daß R² eine Methylgruppe ist, diese E- oder Z-ständig sein kann,
und
- R³: Methyl, Ethyl, Propyl, Isopropyl oder n-Butyl
bedeuten,
in Gegenwart von Lipasen, beispielsweise aus Schweineleber, Schweinepankreas oder aus Mikroorganismen, wie Mucor, Rhizopus, Penicillium, Aspergillus und vorzugsweise Candida oder Pseudomonas umsetzt mit einem Alkohol der Formel II 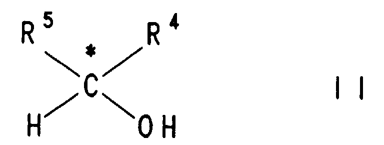 in der
- R⁴: C₁-C₁₈-Alkyl, C₃-C₁₀-Cycloalkyl bedeutet, wobei diese Reste auch halogensubstituiert sein können,
und
- R⁵: Epoxi-C₁-C₅-Alkyl bedeutet, wobei die Epoxi-Gruppe in β-Position zur OH-Gruppe im Rest der Formel II steht
oder
- R⁵: C₁-C₁₀-Alkyl, C₂-C₁₀-Alkenyl, C₂-C₁₀-Alkinyl, C₃-C₈-Cycloalkenyl bedeutet,
wobei die Alkyl-, Alkenyl-, Alkinyl- und Cycloalkenylreste gegebenenfalls mit COOH, Halogen, NO₂, CN, C₁-C₄-Alkoxycarbonyl oder Phenyl substituiert sind, wobei der Phenylrest seinerseits mit Halogen, NO₂, CN oder C₁-C₄-Alkoxy substituiert sein kann, oder R⁴ Aryl oder Heteroaryl bedeutet, wobei die Aryl- oder Heteroarylreste gegebenenfalls mit C₁-C₄-Alkyl, C₁-C₄-Alkoxy, Halogen, NO₂, CN oder N-Sgr. substituiert sind, wobei Sgr. eine Aminoschutzgruppe darstellt,
oder in der
- R⁴ und R⁵: zusammen einen Alkenylen-Rest der Formeln IIIa, b 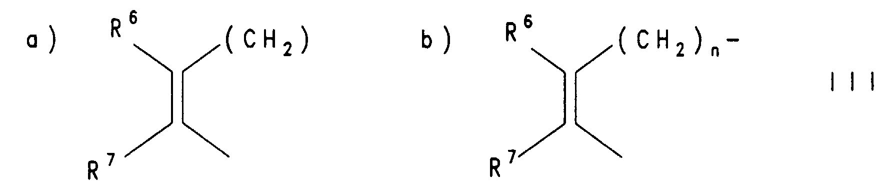
darstellen, in der
- n: 1, 2 oder 3 ist und
R⁶ und R⁷ gleich oder verschieden sind und Wasserstoff, C₂-C₄-Alkenyl, C₁-C₄-Alkyl oder
R⁶ und R⁷ zusammen anelliertes Phenyl oder anelliertes Naphthyl bedeuten, wobei der Phenyl- oder Naphthyl-Rest gegebenenfalls C₁-C₄-Alkyl-, C₁-C₄-Alkoxy-, NO₂-, CN- oder halogensubstituiert ist,
wobei der Alkenylen-Rest der Formel III auch noch eine Ketogruppe aufweisen kann,
oder mit einem Alkohol der Formel IV, 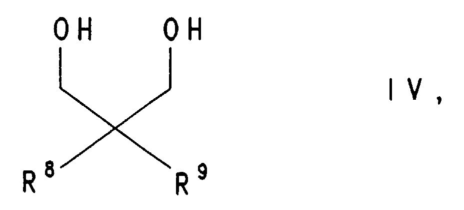 in der
- R⁸: Wasserstoff oder eine Alkylgruppe und
- R⁹: Alkyl, Aralkyl, Aryl, Benzyl, Naphtylmethylgruppe oder O-Alkyl, O-Aralkyl, O-Aryl, O-Benzyl oder O-Naphtylmethyl
bedeuten,
und anschließend den optisch reinen Alkohol isoliert und gegebenenfalls das andere Enantiomere aus dem entstandenen Ester gewinnt.

Vorzugsweise sollte mindestens eine der nachfolgenden Bedingungen erfüllt sein:
- R¹: bedeutet C₁-C₄-Alkyl, welches gegebenenfalls chlorsubstituiert ist;
- R⁴: bedeutet C₁-C₇-Alkyl, welches gegebenenfalls chlorsubstituiert ist, oder R⁴ bedeutet C₃-C₅-Cycloalkyl;
- R⁵: bedeutet C₁-C₄-Alkyl, C₂-C₄-Alkenyl oder C₂-C₄-Alkinyl, wobei die Alkyl-, Alkenyl- oder Alkinylreste gegebenenfalls phenylsubstituiert sind oder
- R⁵: bedeutet Phenyl, Naphthyl, Phenanthryl, Furyl, Thienyl, Pyrrolyl oder Pyridyl, wobei diese Reste gegebenenfalls ihrerseits mit Halogen, NO₂, CN-, C₁-C₄-Alkyl oder C₁-C₄-Alkoxy substituiert sind;
- R⁶ und R⁷: bilden zusammen anelliertes Phenyl.

Bevorzugt kann auch mindestens eine der nachfolgenden Bedingungen erfüllt sein:
- R¹: bedeutet Methyl oder Chlormethyl;
- R⁴: bedeutet C₁-C₅-Alkyl, welches gegebenenfalls chlorsubstituiert ist oder R⁴ bedeutet Cyclopropyl;
- R⁵: bedeutet Phenyl, Naphthyl, Phenanthryl, Furyl oder Pyrridyl, wobei diese Reste gegebenenfalls ihrerseits mit NO₂ oder Methoxy substituiert sind.

Das erfindungsgemäße Verfahren zeichnet sich vorzugsweise dadurch aus, daß Lipasen aus Pseudomonas eingesetzt werden, insbesondere Lipasen aus Pseudomonas cepacia.

Für das erfindungsgemäße Verfahren werden bevorzugt auch Lipasen aus Candida eingesetzt, insbesondere eine Lipase aus Candida antarctica.

Als Ester gemäß Formel I eignen sich insbesondere Methoxy- oder Ethoxyvinylacetat.

Das erfindungsgemäße Verfahren weist gegenüber herkömmlichen Verfahren zur Racematspaltung von Alkoholen folgende Vorteile auf:
a) Zumindest ein Enantiomeres ist immer (optisch) rein, meist sogar beide;
b) das eingesetzte Enzym kann entweder leicht zurückgewonnen oder auch immobilisiert eingesetzt werden;
c) die Trennung des bei der Spaltung entstandenen Esters von dem Alkohol ist sehr einfach; im einfachsten Fall destillative oder chromatographische Trennung;
d) es kann in organischen Lösemitteln oder auch in Substanz gearbeitet werden;
e) hoher und schneller Umsatz bei Verwendung vergleichsweise niedriger Enzymmengen;
f) bei der Acylierung fallen im Gegensatz zur Verwendung von Vinylacetat nur völlig unbedenkliche, untoxische und unreaktive Nebenprodukte an, also beispielsweise Essigsäureethylester beim Einsatz von Ethoxyvinylacetat;
g) die Enzymaktivität ist in manchen Fällen gegenüber Vinylacetat erhöht.
h) Im Vergleich zu Vinylacetat sind die vorliegenden Enolester, bedingt durch die Reste R² bzw. R³, sterisch anspruchsvoller; daraus können sich in manchen Fällen Vorteile durch eine erhöhte Selektivität ergeben.
i) R² und insbesondere R³ können mit chiralen Resten versehen werden. Beispielsweise können bei der Synthese der Enolester statt Ethoxyacetylen die entsprechenden chiralen Acetylene R²-C=C-O-R³* (mit R³** gleich chiral) eingesetzt werden. Daraus ergibt sich die Möglichkeit der doppelten Stereoselektion, also der enzyminduzierten und gleichzeitig der durch die chirale Hilfsgruppe R³* induzierten; große Vorteile können sich somit insbesondere bei den Substraten ergeben, die ansonsten nur eine unbefriedigende enzymatische Stereodifferenzierung erfahren.

Bei dem erfindungsgemäßen Verfahren wird der Ester der Formel I in eine Carbonsäure und den entsprechenden Alkoxyalkenol gespalten. Der Alkoxyalkenol lagert sich sofort in den entsprechenden Alkylester um. Eine Rückreaktion ist somit völlig ausgeschlossen. Unter Katalyse der Enzyme findet auf diese Weise schnell und in hohen Ausbeuten Acylierung statt, die im Fall von racemischen oder prochiralen Substraten hochselektiv ist, d. h. nur ein acyliertes Enantiomer entsteht, das andere verbleibt unverändert im Reaktionsgemisch.

Besonders gut eignet sich das erfindungsgemäße Verfahren zur Spaltung von solchen Alkoholen, die in β-Position zur OH-Gruppe eine C-C-Doppel- oder -Dreifachbindung aufweisen, bzw. die in dieser Position ein Epoxid oder ein Doppelbindungsäquivalent wie einen C₃-Ring besitzen.

Bei dem erfindungsgemäßen Verfahren geht man am besten so vor, daß man den Enolester der Formel I, bevorzugt Ethoxyvinylacetat, in der Lösung eines organischen Lösemittels vorlegt und zu dieser Lösung sowohl Enzym als auch den zu acylierenden Alkohol hinzugibt. Als unpolare organisches Lösemittel verwendet man bevorzugt Ether, wie z. B. Diisopropylether oder t-Butylmethylether oder Kohlenwasserstoffe wie Isohexan, Pentan oder Hexan. Die Konzentration des Enolesters im organischen Lösemittel hängt auch von der Löslichkeit der Substrate ab und kann zwischen 0,05 und 50 % liegen, bevorzugt jedoch zwischen 1 und 10 %. Der zu acylierende Alkohol wird ebenfalls in einer Konzentrationen von 0,05 bis 50 % eingesetzt, bevorzugt jedoch hier 1 bis 20 %. Das molare Verhältnis von Enolester und Alkohol kann bei racemischen Alkoholen bei 0,5 : 1 liegen, d. h. 0,5 Äquivalente zur quantitativen Umsetzung eines Enantiomeren; andererseits kann der Enolester auch in großem Überschuß, z. B. 100 eq., eingesetzt werden, wenn beispielsweise unreaktive Alkohole zu acylieren sind oder aber mit geringen Enzymmengen gearbeitet werden soll. Bevorzugte Mengen liegen hier zwischen 1 und 10 Äquivalenten des Enolesters bezogen auf den Alkohol. Als Enzyme werden bevorzugt Lipasen eingesetzt. Von den Lipasen besonders diejenigen, die käuflich erhältlich sind. Es sind dies insbesondere die Lipoprotein-Lipasen aus Pseudomonas, besonders aus Pseudomonas cepacia (früher P. fluorescens), die beispielsweise unter dem Namen Lipase PS (früher auch P oder FP) der Firma Amano Pharmaceuticals, Nagoya, Japan, im Handel ist. Auch Lipasen aus Candida, besonders Candida rugosa und Candida antarctica (SP 625, Novo Industrie, Bagsvaerd, Dänemark) sind gut geeignet. Das Enzym kann in freier Form oder immobilisiert eingesetzt werden. In beiden Fällen ist auch die Verwendung eines Säulenverfahrens möglich. Die Enzymmenge wird in Abhängigkeit von der Größe des Ansatzes, von der Reaktivität des Alkoholes, von der anzustrebenden Reaktionszeit und von der Art des Enzyms (frei oder fixiert) frei gewählt und ist im Einzelfall durch einfache Vorversuche leicht zu bestimmen.

Die Reaktionstemperatur beträgt -10 °C bis 80 °C, bevorzugt 15 °C bis 40°C. Es ist zweckmäßig, die Lösung während der Reaktion zu rühren. Die Reaktionszeiten schwanken je nach eingesetztem Alkohol und in Abhängigkeit von Enzymmenge und Lösemittel zwischen wenigen Stunden und bis zu 2 Wochen, liegen jedoch zumeist bei einigen Stunden.

Die Enolester sind nicht käuflich, können jedoch leicht aus den entsprechenden Alkoxyacetylenen hergestellt werden (Y. Kita, H. Maeda, K. Omori, T. Okuno, Y. Tamura, J. Chem. Soc. Perkin Trans. 1, 1993, S. 2999-3005). Im Fall des Ethoxyvinylacetats wird z. B. von Ethoxyacetylen ausgegangen. Die Aufreinigung des Enolesters kann z. B. durch Destillation oder Kieselgelchromatographie erfolgen, die Reinheitskontrolle ist leicht durch NMR-Spektroskopie oder GC durchführbar.

Die racemischen, prochiralen oder anderen zu acylierenden Alkohole sind käuflich erhältlich oder nach literaturbekannten Verfahren darstellbar.

Die bei dem erfindungsgemäßen Verfahren entstehenden Produkte lassen sich auf bekannte Art und Weise trennen. Der bei der Acylierung anfallende Essigester im Fall des Ethoxyvinylacetats destilliert leicht ab; Überschuß an Acylierungsreagenz sowie Alkohol und Acylalkohol lassen sich manchmal destillativ, zumeist aber durch einfache Chromatographie oder per Extraktion oder Kristallisation trennen. Aus den acylierten Alkoholen lassen sich die freien Alkohole nach üblichen Methoden freisetzen.

Das in der Reaktion verwendete Enzym läßt sich leicht durch Filtration wiedergewinnen und kann wiederholt verwendet werden.

In den nachfolgenden Beispielen wird die Erfindung im Detail erläutert.

### Beispiel 1:

### Allgemeine Arbeitsvorschrift zur enzymatischen Acylierung mit Ethoxyvinylacetat

Der Alkohol wird in einer 10 % igen Lösung des Enolacetats in Hexan oder t-Butylmethylether gelöst bzw. suspendiert. Dazu gibt man 10 Gewichts-% Lipase PS in freier oder fixierter Form und rührt bei Raumtemperatur. Die Reaktion läßt sich leicht dünnschichtchromatographisch kontrollieren (Laufmittel z. B. Essigester/Hexan in unterschiedlichen Verhältnissen). Nach Beendigung der Reaktion (d. h. vollständiger Acylierung bei einfachen und prochiralen Alkoholen bzw. 50%igem Umsatz im Fall von racemischen Alkoholen) filtriert man das Enzym ab, engt die verbleibende Lösung im Vakuum ein und trennt das als Rückstand verbleibende Alkohol/Ester-Gemisch (falls man einen racemischen Alkohol eingesetzt hat) bevorzugt durch Säulenchromatographie an Kieselgel oder durch Extraktion, Kristallisation oder Destillation.

Statt Lipase PS kann mit ähnlichen Ergebnissen ebenfalls die Lipase SP 625 aus Candida antarctica verwendet werden. Bei einem Einsatz von gleichen Enzymmengen können z. T. etwas schnellere, z. T. aber auch etwas langsamere Umsetzungen festgestellt werden.

### Beispiel 2:

Phenylethanol: 100 mg des Alkohols werden in 2 ml einer 10 % igen Lösung des Ethoxyvinylacetats in Hexan gelöst und mit 10 mg Lipase PS versetzt. Nach ca. 3 h bei RT kann man 50 %igen Umsatz per DC feststellen. Isolierung von Alkohol und Acetat durch Flash-Chromatographie an Kieselgel 60 in Essigester/Hexan (Gradient von 1:10 bis 1:1). Isolierte Ausbeute Acetat 40 %/Alkohol 39 %, ee Acetat > 95 % (¹H-NMR, Shift-Versuch mit Eu(hfc)₃, 10 mg Acetat, 40 mg Shift-Reagenz); Drehwert: + 103,5° (C=1, MeOH)

### Beispiel 3:

1-Butin-3-ol: gleiche Bedingungen wie in Beispiel 2; Reaktionszeit: 24 h; Ausbeute Acetat: 31 %, ee 70 % (¹H-NMR); Alkohol nicht bestimmt.

### Beispiel 4:

Pantolacton: gleiche Bedingungen wie in Beispiel 2; Verwendung von TBME und Reaktionszeit 44 h; Acetat und Alkohol lassen sich trennen (Ausbeute 42 und 41 %) und weisen einen Drehwert auf, der aufgrund von Verunreinigungen nicht genau bestimmt wurde.

### Beispiel 5:

Allethrolon: gleiche Bedingungen wie in Beispiel 2; Reaktionszeit: 10 h; Ausbeute: Acetat 45 %, Alkohol 46 %; ee Acetat 89 % (¹H-NMR, Shift-Versuch wie in Beispiel 2), ee Alkohol nicht bestimmt.

### Beispiel 6:

2-(1-Naphtylmethyl)-propan-1,3-diol: gleiche Bedingungen wie Beispiel 2; Temperatur: 5 °C, Lösemittel Dimethoxyethan/Diisopropylether 1:1 statt Hexan; Reaktionszeit: 10 h, Umsatz 94 %, ee Acetat 94 % (¹H-NMR, Shift-Versuch wie Beispiel 2).

### Beispiel 7:

Geraniol: Bedingungen wie in Beispiel 2; Reaktionszeit 1 h, 35 °C, vollständige Acylierung laut DC.

### Beispiel 8:

Versuch zur nichtenzymatischen Enolesterspaltung: Eine 10 %ige Lösung des Ethoxyvinylacetats in Hexan (2 ml) wird bei RT mit leicht acylierbaren Alkoholen (10 Vol-%) versetzt; ausgewählt wurden Phenylethanol und Allethrolon. Nach 5 h bei RT (21 °C) konnte per DC in beiden Fällen kein Acylierungsprodukt nachgewiesen werden.

### Beispiel 9:

Versuch zur Acylierung mit Essigsäureethylester: Durchführung wie Beispiel 8, nur daß Essigsäureethylester statt Ethoxyvinylacetat verwendet wurde sowie 20 mg Enzym Lipase PS. Acylierungsprodukt konnte nach 2 h per DC nicht nachgewiesen werden.

### Beispiel 10:

Verwendung von Lipase Candida antarctica: Durchführung wie Beispiel 2; 50 % Umsatz werden nach ca. 2 h erreicht.

## Patentansprüche

1. Verfahren zur enzymatischen Acylierung von Alkoholen, dadurch gekennzeichnet, daß man einen Ester der Formel I, 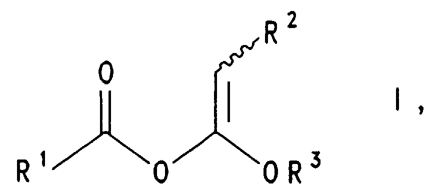 in der
R¹ Wasserstoff, C₁-C₁₈-Alkyl, welches gegebenenfalls mit Halogen substituiert ist, Phenyl oder (C₁-C₃)-Alkoxy (C₁-C₄)-Alkyl und
R² Wasserstoff oder Methyl, wobei für den Fall, daß R² eine Methylgruppe ist, diese E- oder Z-ständig sein kann,
und
R³ Methyl, Ethyl, Propyl, Isopropyl oder n-Butyl
bedeuten,
in Gegenwart von Lipasen aus Schweineleber, Schweinepankreas oder aus Mikroorganismen, wie Candida, Mucor, Rhizopus, Penicillium, Aspergillus und Pseudomonas umsetzt mit einem Alkohol der Formel II 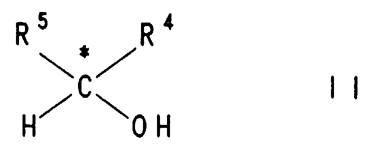 in der
R⁴ C₁-C₁₈-Alkyl, C₃-C₁₀-Cycloalkyl bedeutet, wobei diese Reste auch halogensubstituiert sein können,
und
R⁵ Epoxi-C₁-C₅-Alkyl bedeutet, wobei die Epoxi-Gruppe in β-Position zur OH-Gruppe im Rest der Formel II steht
oder
R⁵ C₁-C₁₀-Alkyl, C₂-C₁₀-Alkenyl, C₂-C₁₀-Alkinyl, C₃-C₈-Cycloalkenyl bedeutet,
wobei die Alkyl-, Alkenyl-, Alkinyl- und Cycloalkenylreste gegebenenfalls mit COOH, Halogen, NO₂, CN, C₁-C₄-Alkoxycarbonyl oder Phenyl substituiert sind, wobei der Phenylrest seinerseits mit Halogen, NO₂, CN oder C₁-C₄-Alkoxy substituiert sein kann, oder R⁵ Aryl oder Heteroaryl bedeutet, wobei die Aryl- oder Heteroarylreste gegebenenfalls mit C₁-C₄-Alkyl, C₁-C₄-Alkoxy, Halogen, NO₂, CN oder N-Sgr. substituiert sind, wobei Sgr. eine Aminoschutzgruppe darstellt,
oder in der
R⁴ und R⁵ zusammen einen Alkenylen-Rest der Formeln IIIa, b 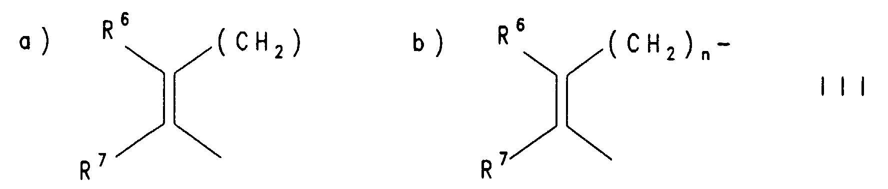
darstellen, in der
n 1, 2 oder 3 ist und
R⁶ und R⁷ gleich oder verschieden sind und Wasserstoff, C₂-C₄-Alkenyl, C₁-C₄-Alkyl oder
R⁶ und R⁷ zusammen anelliertes Phenyl oder anelliertes Naphthyl bedeuten, wobei der Phenyl- oder Naphthyl-Rest gegebenenfalls C₁-C₄-Alkyl-, C₁-C₄-Alkoxy-, NO₂-, CN- oder halogensubstituiert ist,
wobei der Alkenylen-Rest der Formel III auch noch eine Ketogruppe aufweisen kann,
oder mit einem Alkohol der Formel IV, 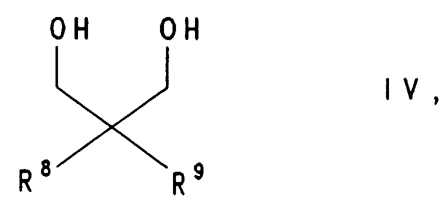 in der
R⁸ Wasserstoff oder eine Alkylgruppe und
R⁹ Alkyl, Aralkyl, Aryl, Benzyl, Naphtylmethylgruppe oder O-Alkyl, O-Aralkyl, O-Aryl, O-Benzyl oder O-Naphtylmethyl
bedeuten,
und anschließend den optisch reinen Alkohol isoliert und gegebenenfalls das andere Enantiomere aus dem entstandenen Ester gewinnt.

2. Verfahren nach Anspruch 1, dadurch gekennzeichnet, daß mindestens eine der nachfolgenden Bedingungen erfüllt ist:
R¹ bedeutet C₁-C₄-Alkyl, welches gegebenenfalls chlorsubstituiert ist;
R⁴ bedeutet C₁-C₇-Alkyl, welches gegebenenfalls chlorsubstituiert ist, oder R⁴ bedeutet C₃-C₅-Cycloalkyl;
R⁵ bedeutet C₁-C₄-Alkyl, C₂-C₄-Alkenyl oder C₂-C₄-Alkinyl, wobei die Alkyl-, Alkenyl- oder Alkinylreste gegebenenfalls phenylsubstituiert sind oder
R⁵ bedeutet Phenyl, Naphthyl, Phenanthryl, Furyl, Thienyl, Pyrrolyl oder Pyridyl, wobei diese Reste gegebenenfalls ihrerseits mit Halogen, NO₂, CN-, C₁-C₄-Alkyl oder C₁-C₄-Alkoxy substituiert sind;
R⁶ und R⁷ bilden zusammen anelliertes Phenyl.

3. Verfahren nach Anspruch 1 oder 2, dadurch gekennzeichnet, daß mindestens eine der nachfolgenden Bedingungen erfüllt ist:
R¹ bedeutet Methyl oder Chlormethyl;
R⁴ bedeutet C₁-C₅-Alkyl, welches gegebenenfalls chlorsubstituiert ist oder R⁴ bedeutet Cyclopropyl;
R⁵ bedeutet Phenyl, Naphthyl, Phenanthryl, Furyl oder Pyrridyl, wobei diese Reste gegebenenfalls ihrerseits mit NO₂ oder Methoxy substituiert sind.

4. Verfahren nach einem der Ansprüche 1 bis 3, dadurch gekennzeichnet, daß Lipasen aus Pseudomonas eingesetzt werden.

5. Verfahren nach Anspruch 4, dadurch gekennzeichnet, daß eine Lipase aus Pseudomonas cepacia eingesetzt wird.

6. Verfahren nach einem der Ansprüche 1 bis 3, dadurch gekennzeichnet, daß Lipasen aus Candida eingesetzt werden.

7. Verfahren nach Anspruch 5, dadurch gekennzeichnet, daß eine Lipase aus Candida antarctica eingesetzt wird.

8. Verfahren nach einem der Ansprüche 1 bis 7, dadurch gekennzeichnet, daß als Ester Methoxy- bzw. Ethoxyvinylacetat eingesetzt wird.

## Claims

1. A process for the enzymatic acylation of alcohols, which comprises reacting an ester of the formula I 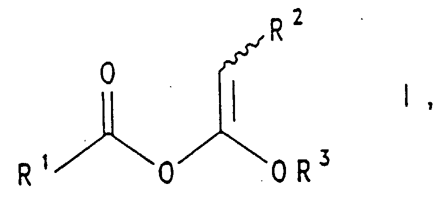 in which
R¹ is hydrogen, C₁-C₁₈-alkyl which is optionally substituted by halogen, or is phenyl or (C₁-C₃)-alkoxy (C₁-C₄)-alkyland
R² is hydrogen or methyl, where if R² is a methyl group this can be in the E- or Z-position,
and
R³ is methyl, ethyl, propyl, isopropyl or n-butyl,
in the presence of lipases from porcine liver, porcine pancreas or from microorganisms, such as Candida, Mucor, Rhizopus, Penicillium, Aspergillus and Pseudomonas, with an alcohol of the formula II 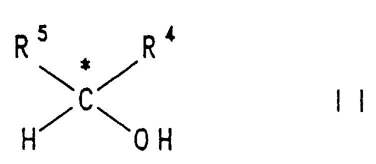 in which
R⁴ is C₁-C₁₈-alkyl or C₃-C₁₀-cycloalkyl, it also being possible for these radicals to be halogen-substituted,
and
R⁵ is epoxy-C₁-C₅-alkyl, the epoxy group being in the β-position to the OH group in the radical of the formula II
or
R⁵ is C₁-C₁₀-alkyl, C₂-C₁₀-alkenyl, C₂-C₁₀-alkynyl or C₃-C₈-cycloalkenyl,
the alkyl, alkenyl, alkynyl and cycloalkenyl radicals optionally being substituted by COOH, halogen, NO₂, CN, C₁-C₄-alkoxycarbonyl or phenyl, it being possible for the phenyl radical, for its part, to be substituted by halogen, NO₂, CN or C₁-C₄-alkoxy, or R⁵ is aryl or heteroaryl, the aryl or heteroaryl radicals optionally being substituted by C₁-C₄-alkyl, C₁-C₄-alkoxy, halogen, NO₂, CN or N-Sgr., where Sgr. is an amino protective group,
or in which
R⁴ and R⁵ together are an alkenylene radical of the formula IIIa or b 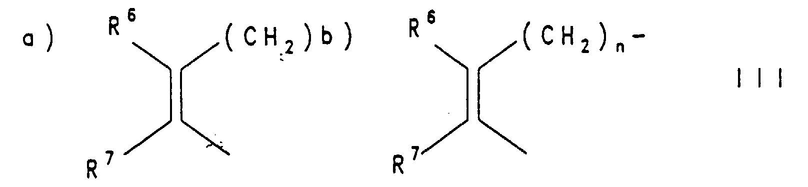 in which
n is 1, 2 or 3 and
R⁶ and R⁷ are identical or different and are hydrogen, C₂-C₄-alkenyl, C₁-C₄-alkyl or
R⁶ and R⁷ together are fused phenyl or fused naphthyl, the phenyl or naphthyl radical optionally being C₁-C₄-alkyl-, C₁-C₄-alkoxy-, NO₂- , CN- or halogen-substituted,
it being possible for the alkenylene radical of the formula III additionally to contain a keto group,
or with an alcohol of the formula IV 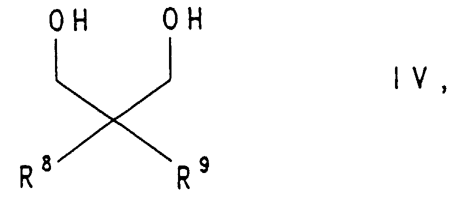 in which
R⁸ is hydrogen or an alkyl group and
R⁹ is alkyl, aralkyl, aryl, benzyl, naphthylmethyl or O-alkyl, O-aralkyl, O-aryl, O-benzyl or O-naphthylmethyl,
and then isolating the optically pure alcohol and optionally recovering the other enantiomer from the ester formed.

2. The process as claimed in claim 1, wherein at least one of the following conditions is fulfilled:
R¹ is C₁-C₄-alkyl, which is optionally chlorine--substituted;
R⁴ is C₁-C₇-alkyl, which is optionally chlorine-substituted, or R⁴ is C₃-C₅-cycloalkyl;
R⁵ is C₁-C₄-alkyl, C₂-C₄-alkenyl or C₂-C₄-alkynyl, the alkyl, alkenyl or alkynyl radicals optionally being phenyl-substituted or
R⁵ is phenyl, naphthyl, phenanthryl, furyl, thienyl, pyrrolyl or pyridyl, these radicals optionally being substituted for their part by halogen, NO₂, CN-, C₁-C₄-alkyl or C₁-C₄-alkoxy;
R⁶ and R⁷ together form fused phenyl.

3. The process as claimed in claim 1 or 2, wherein at least one of the following conditions is fulfilled:
R¹ is methyl or chloromethyl;
R⁴ is C₁-C₅-alkyl, which is optionally chlorine-substituted or R⁴ is cyclopropyl;
R⁵ is phenyl, naphthyl, phenanthryl, furyl or pyridyl, these radicals optionally being substituted for their part by NO₂ or methoxy.

4. The process as claimed in one of claims 1 to 3, wherein lipases from Pseudomonas are employed.

5. The process as claimed in claim 4, wherein a lipase from Pseudomonas cepacia is employed.

6. The process as claimed in one of claims 1 -to 3, wherein lipases from Candida are employed.

7. The process as claimed in claim 5, wherein a lipase from Candida antarctica is employed.

8. The process as claimed in one of claims 1 to 7, wherein the ester employed is methoxy- or ethoxyvinyl acetate.

## Revendications

1. Procédé pour l'acylation enzymatique des alcools, caractérisé en ce que l'on acyle un ester de formule I 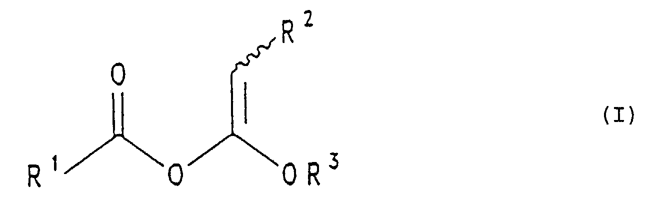 dans laquelle
R¹ représente un atome d'hydrogène, un groupe alkyle en C₁-C₁₈, lequel peut être éventuellement substitué par des atomes d'halogène, des groupes phényle ou (alcoxy en C₁-C₃)-(alkyle en C₁-C₄) et
R² représente un atome d'hydrogène ou un groupe méthyle, pour le cas où R² représente un groupe méthyle, celui-ci pouvant être en position E ou Z, et
R³ représente des groupes méthyle, éthyle, propyle, isopropyle ou n-butyle,
en présence de lipases du foie de porc, du pancréas de porc ou des microorganismes tels que *Candida, Mucor, Rhizopus, Penicillium, Aspergillus* ou *Pseudomonas,* avec un alcool de formule II 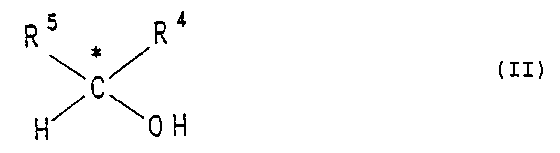 dans laquelle
R⁴ représente des groupes alkyle en C₁-C₁₈, cycloalkyle en C₃-C₁₀, ces restes pouvant être aussi substitués par des halogènes,
et
R⁵ représente époxy(alkyle en C₁-C₅), le groupe époxy étant en position β par rapport au groupe OH dans le reste de formule II,
ou
R⁵ représente des groupes alkyle en C₁-C₁₀, alcényle en C₂-C₁₀, alcynyle en C₂-C₁₀, cycloalcényle en C₃-C₈,
les restes alkyle, alcényle, alcynyle et cycloalcényle étant éventuellement substitués par COOH, des atomes d'halogènes, des groupes NO₂, CN, (alcoxy en C₁-C₄)- carbonyle ou phényle, le reste phényle de son côté pouvant être substitué par un atome d'halogène, NO₂, CN ou un groupe alcoxy en C₁-C₄, ou R⁵ représente un reste aryle ou hétéroaryle, des restes aryle ou hétéroaryle étant éventuellement substitués par des groupes alkyle en C₁-C₄, alcoxy en C₁-C₄, des atomes d'halogènes, des groupes NO₂, CN ou N-Sgr., Sgr représentant un groupe protecteur d'amino,
ou dans laquelle
R⁴ et R⁵ ensemble représentent un reste alcénylène de formules IIIa, IIIb 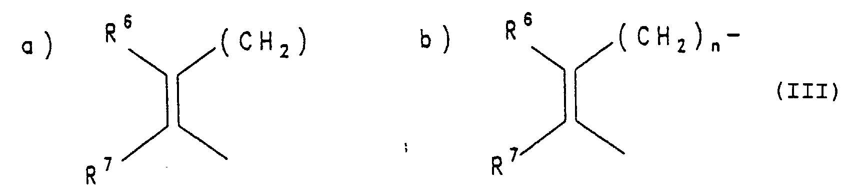 dans laquelle
n vaut 1, 2 ou 3, et
R⁶ et R⁷ sont identiques ou différents et représentent un atome d'hydrogène, des groupes alcényle en C₂-C₄, alkyle en C₁-C₄, ou
R⁶ et R⁷ ensemble représentent un phényle condensé ou un naphtyle condensé, le reste phényle ou naphtyle pouvant être éventuellement substitués par des groupes alkyle en C₁-C₄, alcoxy en C₁-C₄, NO₂, CN ou des atomes d'halogènes,
le reste alcénylène de formule III pouvant présenter encore un groupe céto,
ou avec un alcool de formule IV, 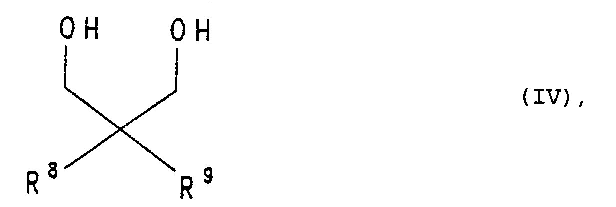 dans laquelle
R⁸ représente un atome d'hydrogène ou un groupe alkyle et
R⁹ représente des groupes alkyle, aralkyle, aryle, benzyle, naphtylméthyle ou O-alkyle, O-aralkyle, O-aryle, O-benzyle ou O-naphtylméthyle,
et on isole ensuite l'alcool optiquement pur et on obtient éventuellement l'autre énantiomère à partir de l'ester formé.

2. Procédé selon la revendication 1, caractérisé en ce que au moins l'une des conditions ci-après est remplie :
R¹ représente un groupe alkyle en C₁-C₄, lequel est éventuellement substitué par du chlore ;
R⁴ représente un groupe alkyle en C₁-C₇, lequel est éventuellement substitué par du chlore, ou R⁴ représente cycloalkyle en C₃-C₅ ;
R⁵ représente des groupes alkyle en C₁-C₄, alcényle en C₂-C₄ ou alcynyle en C₂-C₄, les groupes alkyle, alcényle ou alcynyle pouvant être éventuellement substitués par phényle ou
R⁵ représente des groupes phényle, naphtyle, phénanthryle, furyle, thiényle, pyrrolyle ou pyridyle, ces restes pouvant être éventuellement substitués de leur côté par des atomes d'halogènes, des groupes NO₂, CN, alkyle en C₁-C₄ ou alcoxy en C₁-C₄ ;
R⁶ et R⁷ forment ensemble un phényle condensé.

3. Procédé selon la revendication 1 ou 2, caractérisé en ce que au moins l'une des conditions ci-après est remplie :
R¹ représente un groupe méthyle ou chlorométhyle ;
R⁴ représente un groupe alkyle en C₁-C₅, lequel peut être éventuellement substitué par du chlore, ou R⁴ représente un groupe cyclopropyle ;
R⁵ représente des groupes phényle, naphtyle, phénanthryle, furyle ou pyridyle, ces restes, de leur côté, pouvant être éventuellement substitués par des groupes NO₂ ou méthoxy.

4. Procédé selon l'une des revendications 1 à 3, caractérisé en ce que l'on utilise des lipases de *Pseudomonas.*

5. Procédé selon la revendication 4, caractérisé en ce que l'on utilise une lipase de *Pseudomonas cepacia.*

6. Procédé selon l'une des revendications 1 à 3, caractérisé en ce que l'on utilise des lipases de *Candida.*

7. Procédé selon la revendication 5, caractérisé en ce que l'on utilise une lipase de *Candida antarctica.*

8. Procédé selon une ou plusieurs des revendications 1 à 7, caractérisé en ce que l'on utilise en tant qu'ester, l'acétate de méthoxy-, respectivement d'éthoxyvinyle.
